# EUROPEAN PATENT APPLICATION

(11) **EP 3 845 205 A1**
(43) Date of publication of application: **07.07.2021**
(21) Application number: 20217733.3
(22) Date of filing: 30.12.2020
(51) Int. Cl.: A61F 2/38, A61F 2/30

(54) **FULL KNEE JOINT PROSTHESIS**

(30) Priority: 31.12.2019 CN 201911420053; 09.04.2020 CN 202010274036
(71) Applicant: Beijing Lidakang Technology Co., Ltd, Beijing 101399 (CN)
(72) Inventor: Zhang, Tianyu, Beijing, 101399 (CN); Zhang, Yapeng, Beijing, 101399 (CN); Ma, Shuqin, Beijing, 101399 (CN)
(74) Representative: Groth & Co. KB

(57) **Abstract**

The present invention provides a full knee joint prosthesis which comprises a femur condyle prosthesis part (1) and a shin bone pad prosthesis part (2), the femur condyle prosthesis part is mounted on the shin bone pad prosthesis part.

## Description

### Technical Field

The present invention relates to the field of knee joint prostheses, and particularly relates to a full knee joint prosthesis.

### Background

Currently, the existing artificial knee joint prostheses may cause postoperative adverse reactions, of which those related to patella account for a very large proportion, for example, patella pain, patella snap, patella instability, etc. The main reason is because the design of the prostheses cannot meet differences among individuals, too wide a patella slide groove tends to cause patella instability, and too narrow a patella slide groove tends to cause patella pain or insufficient buckling function. When a femur condyle part matches different models of shin bone pad prosthesis parts, a situation that the femur condyle arc and the shin bone pad prosthesis do not match each other very well often occurs. If such a situation of poor matching between the articular surfaces of femur and shin occurs, wear of the shin bone pad prosthesis part relative to the femur condyle part would increase significantly, and consequently, deposition of a large amount of polyethylene fine particles would fill the interface between the knee joint prosthesis and the condyle, which is an important reason that causes osteolysis and leads to loosening of the prosthesis.

### Summary of the Invention

An objective of the present invention is to provide a full knee joint prosthesis to solve the problem of prior art that an implanted prosthesis has poor stability, is prone to wear and has poor matching of articular surfaces thereof.

In order to realize the above objective, the present invention provides the following technical solutions:
A full knee joint prosthesis, comprising a femur condyle prosthesis part and a shin bone pad prosthesis part, the femur condyle prosthesis part is mounted on the shin bone pad prosthesis part.

In embodiments, a patella groove is disposed on an outer side of the femur condyle prosthesis part, and a femur condyle articular surface is disposed on a bottom of the femur condyle prosthesis part; the patella groove has a depth gradually increasing along a downward extending direction from a top of the outer side of the femur condyle prosthesis part.

In embodiments, the femur condyle prosthesis part is selected from a series of prosthesis parts suitable for different groups of people, the femur condyle articular surfaces of the series of prosthesis parts have different curvature radii and different central distances.

In embodiments, the femur condyle prosthesis part comprises a front condyle, a femur condyle distal end and two rear condyles that are integrally connected.

In embodiments, the femur condyle prosthesis part has a prosthesis flange extending outward to form a prosthesis border.

In embodiments, the curvature radii of the femur condyle articular surfaces of the series of prosthesis parts change gradually according to respective serial numbers of the series of prosthesis parts.

In embodiments, an intercondylar box having a small area is formed between the femur condyle distal end and the two rear condyles, the intercondylar box protrudes outward between the femur condyle distal end and the rear condyles.

In embodiments, a reversed-U shaped shallow groove is provided on an upper half of an inner side of the front condyle, and two L-shaped shallow grooves are symmetrically provided on a lower half of the inner side of the front condyle.

In embodiments, a femur condyle fixing pillar is provided on the femur condyle distal end; and two U-shaped grooves having opposing U-mouths are provided around the femur condyle fixing pillar on the femur condyle distal end.

In embodiments, a shin bone pad prosthesis articular surface is disposed on the shin bone pad prosthesis part, the shin bone pad prosthesis part is selected from a series of prosthesis parts suitable for different groups of people, the shin bone pad prosthesis articular surfaces of the series of prosthesis parts have different curvature radii and different central distances.

The above technical solutions of the present invention have the following beneficial effects:
(1) As for the femur condyle prosthesis part provided by the present embodiments, when buckling, there is a large contact area between the patella groove and the patella bone, which reduces intensity of pressure and wear of polyethylene, so as to improve stability of the groove and the patella bone; the groove can meet differences among different individuals, and as the groove has a gradually deepening depth along with the buckling process, it can provide a sufficiently stable constraint environment for the patella bone, which not only takes the differences among different patients into consideration, but also takes the stability in the movement process of the patella bone into consideration, taking account of both; the adjacent models in the series of femur condyle prosthesis parts of the present embodiments have similar curvature radii and central distances, so that the femur condyle articular surfaces of the adjacent models have shapes that are similar to the greatest extent, which effectively reduces wear between the femur condyle prosthesis part and the shin bone pad prosthesis part when the model of the femur condyle prosthesis part does not match the model of the shin bone pad prosthesis part, and thus reduces the probability of prosthesis loosening; the intercondylar box having a small area provided by the femur condyle prosthesis part of the present embodiments can reserve more bone mass of the patient; the intercondylar box of the femur condyle prosthesis part of the present embodiments has an intercondylar box flange around it, so that, when other components are mounted in the intercondylar box, excessive friction can be avoided, and dislocation caused by excessive friction can be prevented; the prosthesis flange at the prosthesis border of the femur condyle prosthesis part provided by the present embodiments forms different prosthesis flange shapes according to different portions of the prosthesis, so that the osteotomy surface is better fitted, and postoperative discomfort or dysfunction caused by prosthesis exposure can be minimized.
(2) The shin bone pad prosthesis part provided by the present embodiments can reduce wear between the femur condyle prosthesis part and the shin bone pad prosthesis part, and thus reduce the probability of prosthesis loosening.
(3) The full knee joint prosthesis provided by the present embodiments can meet differences among individuals, the rear condyles of the femur condyle prosthesis part thereof satisfies the buckling function of human body, and is stably connected to the patella bone; the femur condyle prosthesis part thereof and the shin bone pad prosthesis part thereof has a high matching degree, which prevents mismatch between the femur condyle articular surface and the shin bone pad prosthesis articular surface, reduces wear between the femur condyle prosthesis part and the shin bone pad prosthesis part, and thus minimizes loosening of the prosthesis. The adjacent models in the series of femur condyle prosthesis parts of the present embodiments have similar curvature radii and central distances, so that the femur condyle articular surfaces of the adjacent models have shapes that are similar to the greatest extent, which effectively reduces wear between the femur condyle prosthesis part and the shin bone pad prosthesis part when the model of the femur condyle prosthesis part does not match the model of the shin bone pad prosthesis part, and thus reduces the probability of prosthesis loosening.

### Brief Description of the Drawings

FIG. 1 is a front view of a femur condyle prosthesis part of the present invention;
FIG. 2 is an A-A sectional view of the femur condyle prosthesis part of the present invention;
FIG. 3 is a B-B sectional view of the femur condyle prosthesis part of the present invention;
FIG. 4 is a C-C sectional view of the femur condyle prosthesis part of the present invention;
FIG. 5 is a D-D sectional view of the femur condyle prosthesis part of the present invention;
FIG. 6 is an E-E sectional view of the femur condyle prosthesis part of the present invention;
FIG. 7 is a left view of the femur condyle prosthesis part of the present invention;
FIG. 8 is a right view of the femur condyle prosthesis part of the present invention;
FIG. 9 is a structural schematic view of the femur condyle prosthesis part of the present invention;
FIG. 10 is a structural schematic view of a shin bone pad prosthesis part of the present invention.

### Reference signs:

1-femur condyle prosthesis part; 11-front condyle; 111-intercondylar box front flange; 112-reversed-U shaped shallow groove; 113-L-shaped shallow groove; 12-femur condyle distal end; 121-femur condyle fixing pillar; 122-U-shaped groove; 13-rear condyle; 14-crossbeam; 15-intercondylar box; 17-patella groove; 18-prosthesis flange; 19-femur condyle articular surface; 2-shin bone pad prosthesis part; 21-shin bone pad prosthesis articular surface; 3-patella bone.

### Detailed Description of Embodiments

In order to make the technical problem to be solved, the technical solutions and the advantages of the present invention clearer, hereinafter, detailed description is given in conjunction with the appended drawings and specific embodiments.

As for the problem with the prior art that an implanted prosthesis has poor stability, is prone to wear and has poor matching of articular surfaces thereof, the present invention provides a full knee joint prosthesis.

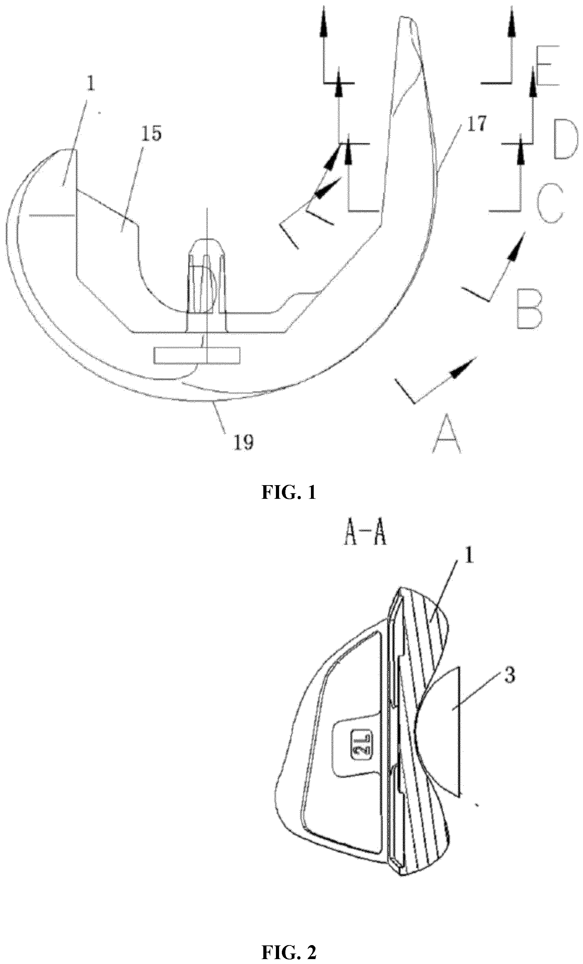

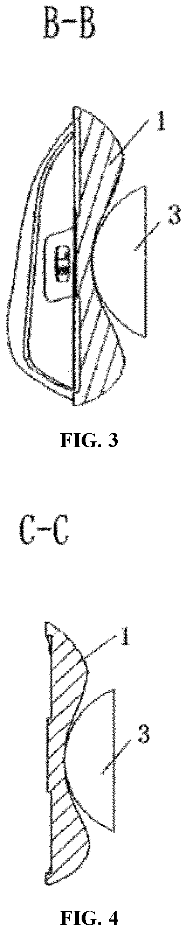

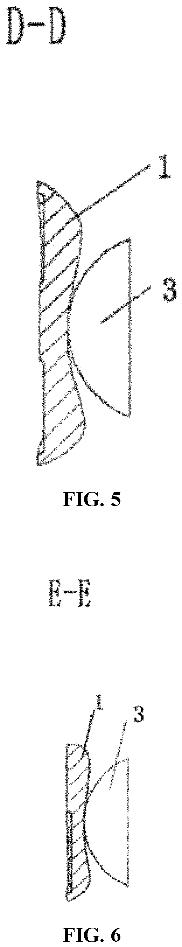

As shown in FIG. 1 to FIG. 10, the present embodiment provides a full knee joint prosthesis which comprises a femur condyle prosthesis part 1 and a shin bone pad prosthesis part 2. The full knee joint prosthesis comprising the femur condyle prosthesis part 1 and the shin bone pad prosthesis part 2 of the present embodiment can meet differences among individuals, the rear condyles 13 of the femur condyle prosthesis part 1 satisfies the buckling function of human body, and is stably connected to the patella bone 3. The femur condyle prosthesis part 1 and the shin bone pad prosthesis part 2 has a high matching degree, which prevents mismatch between the femur condyle articular surface 19 and the shin bone pad prosthesis articular surface 21, reduces wear between the femur condyle prosthesis part 1 and the shin bone pad prosthesis part 2, and thus minimizes loosening of the prosthesis. Specifically, the shin bone pad prosthesis part is provided with an upright column which passes through the intercondylar box 15 of the femur condyle prosthesis part and is brought into contact with the crossbeam 14, and the femur condyle articular surface is brought into contact with the shin bone pad prosthesis articular surface.

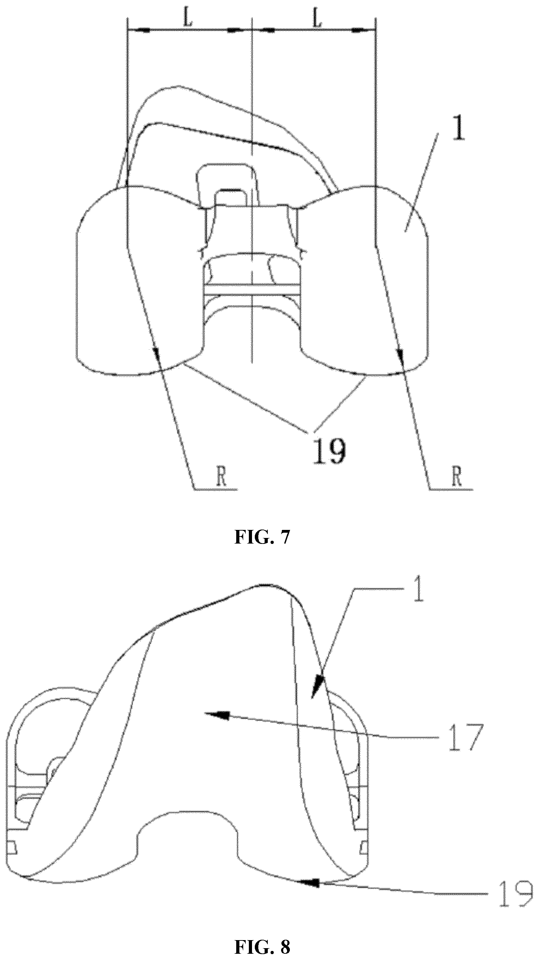

As shown in FIG. 1 to FIG. 8, wherein FIG. 1 is a front view of the present embodiment, FIG. 2 to FIG. 6 are sectional views at five locations of the present embodiment, FIG. 7 and FIG. 8 are respectively left and right views of the femur condyle prosthesis part 1, the present embodiment provides a femur condyle prosthesis part 1, a patella groove 17 is disposed on an outer side of the femur condyle prosthesis part 1, and a femur condyle articular surface 19 is disposed on a bottom of the femur condyle prosthesis part 1; the patella groove 17 has a depth gradually increasing along a downward extending direction from a top of the outer side of the femur condyle prosthesis part 1. The patella groove 17 of the femur condyle prosthesis part 1 provided by the present embodiment is connected to a patella bone 3, and this groove extends gradually from the top of the femur condyle prosthesis part 1 towards the bottom of the femur condyle prosthesis part 1 until it extends to the femur condyle articular surface. The width of this groove, as well as the depth of this groove, gradually increases from the top of the femur condyle prosthesis part 1 to the bottom of the femur condyle prosthesis part 1, so that a groove which gradually widens and deepens starting from the top of the femur condyle prosthesis part 1 is formed, the specific structure thereof is shown in FIG. 2 to FIG. 6. In addition, a contact area between the groove and the patella bone 3 becomes increasingly larger from the top of the femur condyle prosthesis part 1, so that, during a buckling process of human body, a large contact area is kept between the groove and the patella bone 3, which reduces intensity of pressure and wear of polyethylene, so as to improve stability of the groove and the patella bone 3. This groove can meet differences among different individuals, and as the groove gradually deepens along with the buckling process, it can provide a sufficiently stable constraint environment for the patella bone 3, which not only takes the differences among different patients into consideration, but also takes the stability in the movement process of the patella bone 3 into consideration. As a result, the stability during buckling can be improved.

The curvature radii and central distances of the articular surfaces of the femur condyle prosthesis parts 1 of the present embodiment is listed in Table 1-1, including curvature radii and central distances of different models of a series of prosthesis parts. FIG. 7 is a left view of one femur condyle prosthesis part 1 of the present embodiment, wherein, **R** represents a radius of the articular surface of the femur condyle prosthesis part 1, and **L** represents a central distance of the articular surface of the femur condyle prosthesis part. The femur condyle prosthesis parts 1 provided by the present embodiment include a series of prosthesis parts suitable for different groups of people, the femur condyle articular surfaces 19 of the series of prosthesis parts have different curvature radii and different central distances. Specifically, the series of prosthesis parts provided by the present embodiment includes 7 models, the curvature radius and central distance of each model of prosthesis part is different from those of other models. Furthermore, the curvature radii of the femur condyle articular surfaces 19 of the series of prosthesis parts change gradually according to respective serial numbers of the series of prosthesis parts, and the central distances thereof are adapted to the curvature radii, the arrangement of curvature radii and central distances of the series of femur condyle prosthesis parts 1 can effectively reduce wear between the femur condyle prosthesis part 1 and the shin bone pad prosthesis part 2. When implanting a prosthesis, different models of femur condyle prosthesis parts 1 can be selected according to differences among individuals. Even if the model of the femur condyle prosthesis part 1 does not match the model of the shin bone pad prosthesis part 2, because the adjacent models in the series of femur condyle prosthesis parts 1 of the present application have similar curvature radii and central distances, the femur condyle articular surfaces 19 of the adjacent models have shapes that are similar to the greatest extent, so that wear between the femur condyle prosthesis part 1 and the shin bone pad prosthesis part 2 when the model of the femur condyle prosthesis part does not match the model of the shin bone pad prosthesis part can be effectively reduced, thereby reducing the probability of prosthesis loosening.

**Table 1-1**

| | 1# | 2# | 3# | 4# | 5# | 6# | 7# |
|---|---|---|---|---|---|---|---|
| curvature radius | 21 | 22 | 22.5 | 24 | 25.5 | 25.7 | 26 |
| central distance | 21 | 21.5 | 22 | 22.7 | 23 | 23.6 | 24 |

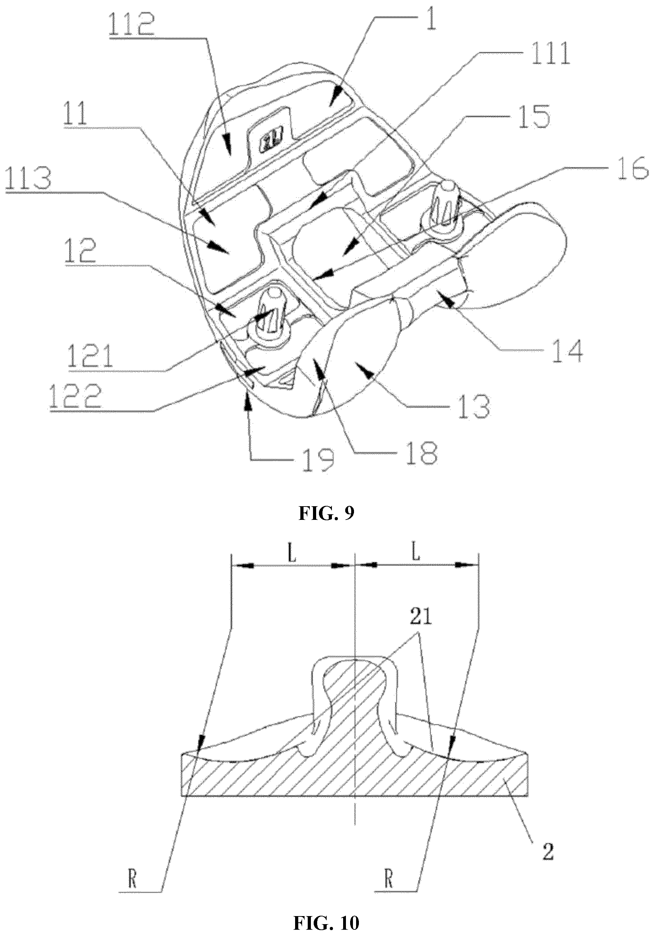

As shown in FIG. 9 which is a structural schematic view of the femur condyle prosthesis part 1 of the present embodiment, the femur condyle prosthesis part 1 provided by the present embodiment comprises a front condyle 11, a femur condyle distal end 12 and two rear condyles 13 that are integrally connected. Furthermore, an intercondylar box 15 having a small area is formed between the femur condyle distal end 12 and the two rear condyles 13, the intercondylar box protrudes outward between the femur condyle distal end 12 and the rear condyles 13. The intercondylar box 15 having a small area formed between the femur condyle distal end 12 and the two rear condyles 13 can reserve more bone mass of the patient. Specifically, the intercondylar box 15 has a near-rectangular shape, a relatively small intercondylar box front flange 111 is arranged at the connection to the femur condyle distal end 12, one end of the intercondylar box 15 is located at the intercondylar box front flange 111, the intercondylar box 15 of the present embodiment is formed by directly slotting the femur condyle distal end 12, so the intercondylar box 15 has a small shape and a small depth, thereby forming an intercondylar box 15 with a small area. The intercondylar box 15 protrudes outward between the femur condyle distal end 12 and the rear condyles 13, the intercondylar box 15 can provide a certain friction interface when the shin bone pad prosthesis part 2 is brought into contact with the intercondylar structure, so that the intercondylar upright column 31 of the shin bone pad prosthesis part 2 is not in direct contact with the bone surface, excessive friction thereof can be avoided, and dislocation caused by excessive friction can be prevented. Specifically, an intercondylar box flange is provided around the intercondylar box 15, the intercondylar box flange is an upward protrusion along the edge of the intercondylar box 15, the intercondylar box 15 has a shallow depth, and as the femur condyle prosthesis part 1 is implemented to be a metal femur condyle prosthesis part 1, arranging the femur condyle prosthesis part 1 to have an intercondylar box 15 with a small area can reserve more bone mass of the patient. The intercondylar box 15 has the intercondylar box flange around it, so that, when other components are mounted in the intercondylar box, excessive friction thereof can be avoided, and dislocation caused by excessive friction can be prevented.

As shown in FIG. 9, the femur condyle prosthesis part 1 provided by the present embodiment has a prosthesis flange extending outward to form a prosthesis border. The femur condyle prosthesis part provided by the present embodiment has a prosthesis flange 18 extending outward to form a prosthesis border. The width of the portion of prosthesis flange 18 on the front condyle 11 gradually increases along an extending direction from the top of the front condyle 11 to the femur condyle distal end 12. The portion of prosthesis flange 18 on the femur condyle distal end 12 has an approximately uniform width. The width of the portion of prosthesis flange 18 on one rear condyle 13 is larger than the width of the portion of prosthesis flange 18 on the other rear condyle 13, and a circular arc transition connection is formed between the portion of prosthesis flange 18 with a larger width on one rear condyle 13 and the portion of prosthesis flange 18 on the femur condyle distal end 12. As the prosthesis flange at the prosthesis border forms prosthesis flange portions of different shapes according to different portions of the prosthesis, so that the osteotomy surface is better fitted, and postoperative discomfort or dysfunction caused by prosthesis exposure can be minimized.

As shown in FIG. 9, a reversed-U shaped shallow groove is provided on an upper half of an inner side of the front condyle 11 provided by the present embodiment, and two L-shaped shallow grooves are symmetrically provided on a lower half of the inner side of the front condyle 11. The front condyle 11 provided by the present embodiment has a reversed-U shaped shallow groove 112 on the upper half of the inner side thereof, and has two L-shaped shallow grooves 113 symmetrically arranged on the lower half of the inner side thereof, thus, the reversed-U shaped shallow groove 112, the L-shaped shallow grooves 113 and the portion of prosthesis flange 18 on the front condyle 11 together form recessed areas that can well fix the bone. Femur condyle fixing pillars 121 are provided on the femur condyle distal end 12 provided by the present embodiment, and two U-shaped grooves 122 having opposing U-mouths are provided around each femur condyle fixing pillar 121 on the femur condyle distal end 12. Each of the femur condyle fixing pillars 121 on the femur condyle distal end 12 has two U-shaped grooves 122 with opposing U-mouths arranged around it, thus, the U-shaped grooves 122 and the portion of prosthesis flange 18 near the femur condyle fixing pillars 121 form recessed areas that can effectively fix the bone.

As shown in FIG. 10 which is a structural schematic view of a shin bone pad prosthesis part 2 of the present embodiment, the curvature radii and central distances of the shin bone pad prosthesis articular surfaces 21 of the shin bone pad prosthesis parts 2 of the present embodiment is listed in Table 1-2. The present embodiment provides a shin bone pad prosthesis part 2, a shin bone pad prosthesis articular surface 21 is disposed on the shin bone pad prosthesis part 2, the shin bone pad prosthesis part 2 is selected from a series of prosthesis parts suitable for different groups of people, the shin bone pad prosthesis articular surfaces 21 of the series of prosthesis parts have different curvature radii and different central distances. When implanting a prosthesis, different models of shin bone pad prosthesis parts 2 can be selected according to differences among individuals. Even if the model of the shin bone pad prosthesis part 2 does not match the model of the femur condyle prosthesis part 1, because the adjacent models in the series of shin bone pad prosthesis parts 2 of the present application have similar curvature radii and central distances, the shin bone pad prosthesis articular surface 21 of the adjacent models have shapes that are similar to the greatest extent, so that wear between the femur condyle prosthesis part 1 and the shin bone pad prosthesis part 2 when the model of the shin bone pad prosthesis part 2 does not match the model of the femur condyle prosthesis part 1 can be effectively reduced, thereby reducing the probability of prosthesis loosening.

**Table 1-2**

| | 1# | 2# | 3# | 4# | 5# | 6# | 7# |
|---|---|---|---|---|---|---|---|
| curvature radius | 21.3 | 21.6 | 22.1 | 22.6 | 23 | 23.6 | 24 |
| central distance | 21 | 22 | 22.5 | 24 | 25.5 | 25.7 | 26 |

In the above technical solutions:
(1) As for the femur condyle prosthesis part 1 provided by the present embodiments, when buckling, there is a large contact area between the patella groove 17 and the patella bone 3, which reduces intensity of pressure and wear of polyethylene, so as to improve stability of the groove and the patella bone 3; the groove can meet differences among different individuals, and as the groove has a gradually deepening depth along with the buckling process, it can provide a sufficiently stable constraint environment for the patella bone 3, which not only takes the differences among different patients into consideration, but also takes the stability in the movement process of the patella bone 3 into consideration; the adjacent models in the series of femur condyle prosthesis parts 1 of the present embodiments have similar curvature radii and central distances, so that the femur condyle articular surfaces 19 of the adjacent models have shapes that are similar to the greatest extent, which effectively reduces wear between the femur condyle prosthesis part 1 and the shin bone pad prosthesis part 2 when the model of the femur condyle prosthesis part 1 does not match the model of the shin bone pad prosthesis part 2, and thus reduces the probability of prosthesis loosening; the intercondylar box having a small area provided by the femur condyle prosthesis part 1 of the present embodiments can reserve more bone mass of the patient; the intercondylar box of the femur condyle prosthesis part 1 of the present embodiments has an intercondylar box flange around it, so that, when other components are mounted in the intercondylar box, excessive friction can be avoided, and dislocation caused by excessive friction can be prevented; the prosthesis flange at the prosthesis border of the femur condyle prosthesis part 1 provided by the present embodiments forms different prosthesis flange shapes according to different portions of the prosthesis, so that the osteotomy surface is better fitted, and postoperative discomfort or dysfunction caused by prosthesis exposure can be minimized.
(2) The shin bone pad prosthesis part 2 provided by the present embodiments can reduce wear between the femur condyle prosthesis part and the shin bone pad prosthesis part 2, and thus reduce the probability of prosthesis loosening.
(3) The full knee joint prosthesis provided by the present embodiments can meet differences among individuals, the patella groove 17 of the femur condyle prosthesis part 1 thereof satisfies the buckling function of human body, and is stably connected to the patella bone 3; the femur condyle prosthesis part 1 thereof and the shin bone pad prosthesis part 2 thereof has a high matching degree, which prevents mismatch between the femur condyle articular surface 19 and the shin bone pad prosthesis articular surface 21, reduces wear between the femur condyle prosthesis part 1 and the shin bone pad prosthesis part 2, and thus minimizes loosening of the prosthesis. The adjacent models in the series of femur condyle prosthesis parts 1 of the present embodiments have similar curvature radii and central distances, so that the femur condyle articular surfaces 19 of the adjacent models have shapes that are similar to the greatest extent, which effectively reduces wear between the femur condyle prosthesis part 1 and the shin bone pad prosthesis part 2 when the model of the femur condyle prosthesis part 1 does not match the model of the shin bone pad prosthesis part 2, and thus reduces the probability of prosthesis loosening.

The embodiments in the present specification are described in a progressive manner, the differences between one embodiment and another embodiment are described and emphasized, while the same or similar parts of the embodiments can refer to one another.

In the description of the present invention, it needs to be understood that, terms such as "above", "one end" refer to the orientation or positional relationship based on the illustration of the appended drawings, and are only for the purpose of facilitating and simplifying the description of the present invention, rather than indicating or implying that the apparatus or component referred to must have a particular orientation or must be configured or operated in a particular orientation, therefore should not be construed as a limitation towards the present invention.

In the description of the present invention, it needs to be noted that, unless specifically defined or restricted otherwise, terms such as "provided", "connected" should be broadly construed, for example, they may be fixed connection or detachable connection or integral connection; they may be direct connection, or indirect connection via an intermediate medium. Said fixed connection may be by ordinary technical means such as welding, threaded connection, or snap-fitting. For a person with ordinary skill in the art, the specific meaning of the aforementioned terms in the present invention can be understood according to specific circumstances thereof.

The above are preferable embodiments of the present invention, and it should be pointed out that, for a person with ordinary skill in the art, on the premise of not departing from the principle of the present invention, various modifications and alterations can be made, and these modifications and alterations should also be embraced within the protection scope of the present invention.

## Claims

1. A full knee joint prosthesis, **characterized in** comprising a femur condyle prosthesis part and a shin bone pad prosthesis part, the femur condyle prosthesis part is mounted on the shin bone pad prosthesis part.

2. The full knee joint prosthesis according to Claim 1, **characterized in that**, a patella groove is disposed on an outer side of the femur condyle prosthesis part, and a femur condyle articular surface is disposed on a bottom of the femur condyle prosthesis part, wherein the patella groove has a depth gradually increasing along a downward extending direction from a top of the outer side of the femur condyle prosthesis part.

3. The full knee joint prosthesis according to Claim 2, **characterized in that**, the femur condyle prosthesis part is selected from a series of prosthesis parts suitable for different groups of people, the femur condyle articular surfaces of the series of prosthesis parts have different curvature radii and different central distances.

4. The full knee joint prosthesis according to Claim 2, **characterized in that**, the femur condyle prosthesis part comprises a front condyle, a femur condyle distal end and two rear condyles that are integrally connected.

5. The full knee joint prosthesis according to Claim 2, **characterized in that**, the femur condyle prosthesis part has a prosthesis flange extending outward to form a prosthesis border.

6. The full knee joint prosthesis according to Claim 3, **characterized in that**, the curvature radii of the femur condyle articular surfaces of the series of prosthesis parts change gradually according to respective serial numbers of the series of prosthesis parts.

7. The full knee joint prosthesis according to Claim 4, **characterized in that**, an intercondylar box having a small area is formed between the femur condyle distal end and the two rear condyles, the intercondylar box protrudes outward between the femur condyle distal end and the rear condyles.

8. The full knee joint prosthesis according to Claim 4, **characterized in that**, a reversed-U shaped shallow groove is provided on an upper half of an inner side of the front condyle, and two L-shaped shallow grooves are symmetrically provided on a lower half of the inner side of the front condyle.

9. The full knee joint prosthesis according to Claim 4, **characterized in that**, a femur condyle fixing pillar is provided on the femur condyle distal end, and two U-shaped grooves having opposing U-mouths are provided around the femur condyle fixing pillar on the femur condyle distal end.

10. The full knee joint prosthesis according to Claim 1, **characterized in that**, a shin bone pad prosthesis articular surface is disposed on the shin bone pad prosthesis part, the shin bone pad prosthesis part is selected from a series of prosthesis parts suitable for different groups of people, the shin bone pad prosthesis articular surfaces of the series of prosthesis parts have different curvature radii and different central distances.
